# EUROPÄISCHE PATENTANMELDUNG

(11) **EP 2 036 537 A1**
(43) Veröffentlichungstag der Anmeldung: **18.03.2009**
(21) Anmeldenummer: 07017804.1
(22) Anmeldetag: 11.09.2007
(51) Int. Cl.: A61K 8/97, A61Q 19/00

(54) **Makroalgenextrakt**

(71) Anmelder: Inwater Biotec GmbH, 24147 Kiel (DE)
(72) Erfinder: Burmeister, Thomas, 24147 Kiel (DE)
(74) Vertreter: Strehl Schübel-Hopf & Partner

(57) **Zusammenfassung**

Die vorliegende Erfindung betrifft einen Makroalgenextrakt, dessen Herstellung und dessen Verwendung als kosmetisches Mittel. Insbesondere betrifft die Erfindung ein Verfahren zur Gewinnung eines Extrakts aus Makroalgen, welches das Behandeln der Makroalgen mit einem Glykol und Wasser bei einer Temperatur von 50 bis 80 °C und das Isolieren des Extrakts umfasst.

## Beschreibung

### Gebiet der Erfindung

Die vorliegende Erfindung betrifft einen Makroalgenextrakt, dessen Herstellung und dessen Verwendung als kosmetisches Mittel.

### Hintergrund der Erfindung

Die Inhaltsstoffe von Makroalgen entsprechen weitgehend denen unserer Körperzellen, so dass sie sehr positive und wirkungsvolle Eigenschaften für den Menschen aufweisen können.

Im Stand der Technik sind jedoch bislang keine Extrakte von Makroalgen bekannt, welche die verwertbaren Inhaltsstoffe der Makroalgen in diesen Konzentrationen enthalten und somit als Kosmetika eingesetzt werden könnten.

Bei herkömmlichen Extraktionsverfahren werden wässrige Auszüge aus Algen gewonnen, die jedoch als Kosmetika ungeeignet sind.

### Durch die Erfindung zu lösende Aufgabe

Der vorliegenden Erfindung liegt die Aufgabe zugrunde, einen Makroalgenextrakt, der als kosmetisches Mittel eingesetzt werden kann, und ein Verfahren zu dessen Herstellung bereitzustellen.

### Lösung der Aufgabe

Die der vorliegenden Erfindung zugrundeliegende Aufgabe wird dadurch gelöst, daß ein Makroalgenextrakt mit Hilfe von Glykolen hergestellt wird.

Genauer gesagt stellt die vorliegende Erfindung ein Verfahren zur Gewinnung eines Makroalgenextrakts bereit, welches das Behandeln der Makroalgen mit einem Glykol und Wasser bei einer Temperatur von 50 bis 80 °C und das Isolieren des Extrakts umfaßt.

Durch das erfindungsgemäße Verfahren kann ein Extrakt erhalten werden, der die wesentlichen Inhaltsstoffe, wie beta-Carotin, Lutein, Polysaccharide (feuchtigkeitsspendende Wirkung), Vitamine A (Anti-Ageing-Eigenschaften), B (Nährstoff für die Zellen) und C (Antioxidans), Kalium, Kalzium, Phosphor, Eisen und alle essentiellen Aminosäuren plus 9 weitere Aminosäuren enthält.

Diese Inhaltsstoffe haben zellaufbauende und feuchtigkeitsspendende bzw. feuchtigkeitshaltende Wirkung. Das Hautbild wird positiv verändert, die Poren werden kleiner, die Haut wird geschmeidiger, die Faltenbildung wird positiv beeinflusst, Couperose und Besenreißer können abgebaut werden. Das bedeutet, dass der Extrakt ausgeprägte Anti-Ageing-Eigenschaften aufweist, ohne bei langfristiger Benutzung Hautirritationen hervorzurufen. Außerdem kann auf Erdölderivate und Konservierungsstoffe, im wesentlichen hier Parabene, die bei längerer Anwendung meist negative Wirkung auf die Haut haben, gänzlich verzichtet werden.

Das in dem erfindungsgemäßen Verfahren eingesetzte Glykol ist vorzugsweise Propylenglykol und Butandiol. Es können jedoch auch andere Glykole verwendet werden.

Nach der Extraktion kann der Auszug mit üblichen Verfahren aufgearbeitet werden. Erfindungsgemäß wird der Extrakt vorzugsweise durch Filtration von den festen Bestandteilen in dem Extraktionsgemisch abgetrennt.

Das Extraktionsgemisch enthält vorzugsweise 100 Gewichtsteile der Makroalgen, 50 bis 300 Gewichtsteile des Glykols und 30 bis 60 Gewichtsteile Wasser.

In einer besonders bevorzugten Ausführungsform des erfindungsgemäßen Verfahrens werden zunächst die getrockneten Makroalgen mit Wasser benetzt, dann wird das Glykol zu den mit Wasser benetzten Algen gegeben, anschließend wird Wasser bis zur endgültigen Menge zugegeben, und das erhaltene Gemisch wird bei der erhöhten Temperatur gerührt.

In dem erfindungsgemäßen Verfahren werden vorzugsweise zunächst 100 Gewichtsteile Makroalgen mit Wasser benetzt, dann werden 200 Gewichtsteile Propylenglykol zugegeben, danach wird Wasser bis zu einer Gesamtmenge von 50 Gewichtsteilen zugegeben, das erhaltene Gemisch wird dann 6 bis 8 Stunden bei 50 bis 80 °C gerührt und anschließend wird der erhaltene Extrakt abfiltriert.

Die vorliegende Erfindung betrifft außerdem einen Extrakt, der durch das erfindungsgemäße Verfahren erhalten werden kann, sowie eine Kapsel, die diesen Extrakt enthält.

Der erfindungsgemäße Extrakt kann als kosmetisches Mittel verwendet werden, beispielsweise als kosmetisches Mittel für die Haut und/oder das Haar in der Form von kosmetischen Salben, Cremes oder Shampoos.

Im folgenden wird ein praktisches Ausführungsbeispiel zur Gewinnung eines erfindungsgemäßen Makroalgenextrakts beschrieben.
1. Die Extraktion wird mit der Zuchtalge Palmaria Palmata Rotalge (Dulse) durchgeführt.
2. Nach der Ernte der Alge wird diese in einem handelsüblichen Kondenstrockner getrocknet.
3. Nach der Trocknung wird die Alge mindestens 4 Tage abgelagert. Dann ist sie für die Extraktion geeignet.
4. Die Extraktion erfolgt in einem temperierbaren Mischer ("Thermomix" der Firma Vorwerk Elektro). Die Alge wird zur Vorbereitung mit hochgereinigtem Wasser gespült und danach unter Zugabe einer glykolischen Verbindung und von hochgereinigtem (Osmose) Wasser in das Gerät eingefüllt (zu 300 g Algen werden 150 g Wasser gegeben). Danach wird der Extraktionsvorgang unter Einstellung des Gerätes auf eine Temperatur von 70°C und permanentem Rühren auf unterster Stufe gestartet. Nach 6 Stunden ist die Extraktion beendet.
5. Nach Beendigung der Extraktion wird der Extrakt mit Hilfe eines Analysesiebes filtriert und ca. 6 Stunden im Filtrierzustand gehalten.
6. Danach wird der Extrakt noch einmal feinfiltriert.
7. Der Extrakt kann dann für die kosmetische Verwendung weiterverarbeitet werden.

Dieses besonders bevorzugte Verfahren unterscheidet sich von anderen Extraktionsverfahren durch den Einsatz einer Zuchtalge, das Trocknungsverfahren und den Einsatz eines temperierbaren Mischers sowie die überwachte Zeitschiene.

## Patentansprüche

1. Verfahren zur Gewinnung eines Extrakts aus Makroalgen, welches das Behandeln der Makroalgen mit einem Glykol und Wasser bei einer Temperatur von 50 bis 80 °C und das Isolieren des Extrakts umfaßt.

2. Verfahren nach Anspruch 1, wobei das Glykol Propylenglykol ist.

3. Verfahren nach Anspruch 1 oder 2, wobei der Extrakt durch Filtration isoliert wird.

4. Verfahren nach einem der vorstehenden Ansprüche, wobei das Extraktionsgemisch 100 Gewichtsteile der Makroalgen, 50 bis 300 Gewichtsteile der Glykol-Verbindungen Propylenglykol und Butandiol und 30 bis 60 Gewichtsteile Wasser enthält.

5. Verfahren nach einem der vorstehenden Ansprüche, wobei während der Extrahierung gerührt wird.

6. Verfahren nach Anspruch 5, wobei mittels einer Vorrichtung mit Rührwerk und Heizung gerührt wird.

7. Verfahren nach einem der vorstehenden Ansprüche, wobei zunächst die getrockneten Makroalgen mit Wasser benetzt werden, das Glykol zu den mit Wasser benetzten Algen gegeben wird, dann Wasser bis zur endgültigen Menge zugegeben wird und das erhaltene Gemisch bei der erhöhten Temperatur gerührt wird.

8. Verfahren nach einem der vorstehenden Ansprüche, wobei 6 bis 8 Stunden gerührt wird.

9. Verfahren nach einem der vorstehenden Ansprüche, wobei zunächst 100 Gewichtsteile Makroalgen mit Wasser benetzt werden, 200 Gewichtsteile Propylenglykol zugegeben werden, dann Wasser bis zu einer Gesamtmenge von 50 Gewichtsteilen zugegeben wird, das erhaltene Gemisch 6 bis 8 Stunden bei 50 bis 80 °C gerührt wird und dann der erhaltene Extrakt abfiltriert wird.

10. Extrakt, erhältlich durch ein Verfahren nach einem der Ansprüche 1 bis 9.

11. Kapsel, die den Extrakt nach Anspruch 10 enthält.

12. Verwendung des Extrakts nach Anspruch 10 als kosmetisches Mittel.

13. Verwendung nach Anspruch 12, wobei das kosmetische Mittel für die Haut und/oder das Haar eingesetzt wird.

14. Verwendung nach Anspruch 12 oder 13, wobei der Extrakt für kosmetische Salben, Cremes oder Shampoos eingesetzt wird.
